# EUROPEAN PATENT SPECIFICATION

(11) **EP 0 297 912 B1**
(45) Date of publication and mention of the grant of the patent: **15.12.1993**
(21) Application number: 88306038.6
(22) Date of filing: 30.06.1988
(51) Int. Cl.: C12N 11/02, G01N 33/546, C22B 3/00, C12P 1/00, C12P 19/24

(54) **Process for immobilization**
Immobilisierungsverfahren
Procédé d'immobilisation

(30) Priority: 01.07.1987 DK 3369/87
(43) Date of publication of application: 04.01.1989
(73) Proprietor: NOVO NORDISK A/S, 2880 Bagsvaerd (DK)
(72) Inventor: Moellgaard, Henrik, DK-2800 Lyngby (DK)
(74) Representative: Knudsen, Sten Lottrup

(56) References cited:
- EP-A- 0 089 165
- EP-A- 0 206 687

## Description

This invention relates to a process for immobilizing biological materials by cross-linking with polyazetidine.

### BACKGROUND OF THE INVENTION

The field of immobilized biologically active materials emerged about twenty years ago, and since then a large number of publications have appeared on immobilization techniques and on the uses of immobilized products. Such studies cover immobilization of enzymes, cells, antibodies and co-enzymes.

Several uses of immobilized products have been commercialized in this period, ranging from the use of glucose isomerase in large industrial plants to the use of immobilized antibodies for purification purposes.

Among the many immobilization methods known in the art, one group of methods is known as cross-linking, as described in K. Mosbach, Methods of Enzymology, 44 (1976). Cross-linking occurs by reacting the material with bi- or polyfunctional cross-linking agents.

Glutaraldehyde has been used as a cross-linking agent both for enzymes (see e.g. DK 143569) and for microbial cells (see e.g. US patent 3,779,869). Yet, glutaraldehyde is not an ideal cross-linking agent, as it only reacts with -NH₂ and -SH groups. Many enzymes and cells react poorly with glutaraldehyde due to a low content of these groups.

A polyazetidine polymer may be employed advantageously for cross-linking purposes, as disclosed in European patent publication EP 0 206 687. The polyazetidine polymer cross-linking system is more widely applicable to immobilization than is glutaraldehyde because it reacts with -COOH, -OH and -NH-groups, as well as -NH₂ and -SH groups.

The immobilization method disclosed in EP 0 206 687 is, however, not ideal in the following respects:

- The pasty consistency of the cell mass at the time of mixing with the polyazetidine polymer makes the mixing process difficult.
- The amount of cross-linking agents is fairly large, generally 5-30% of polyazetidine and 5-40% of glutaraldehyde, and preferably 10-20% of each (% of cell sludge dry matter). We believe that large amounts become necessary because of poor mixing with polyazetidine due to the pasty consistency at the time of mixing.
- The resulting particles are very dense with high diffusion limitation, probably due to the big amount of cross-linking agents.

Immobilization with polyazetidine is also described in G.J. Calton et al., Biotechnology, vol. 4, pp. 317-320 (1986), in US patent 4,436,813, in Wood et al., Bio/Technology, December 1984, pp. 1081-1084, and in European patent publication EP 0 089 165. However, these are not pure cross-linking methods, but instead involve attachment of active cells to a carrier. Carrier bound immobilization is in many cases undesirable as the use of a carrier dilutes the activity leading to low-activity products, and an expensive carrier material is often required.

### OBJECT OF THE INVENTION

It is an object of the invention to overcome or at least mitigate the above mentioned difficulties by providing a process which can be used to prepare immobilized biological materials with high activity and high physical strength by cross-linking with polyazetidine.

### STATEMENT OF THE INVENTION

The invention provides a process for immobilizing biological material by cross-linking with polyazetidine, which process comprises the sequential steps of aqueously mixing biological material with polyazetidine, flocculating the resultant mixture, dewatering the flocculated product, sub-dividing the dewatered product and then curing the sub-divided material to obtain a particle form immobilized product. In this process the cross-linking reaction occurs mainly during the final curing.

The particle-form immobilized product prepared according to the invention is well suited for use in packed-bed columns, where it exhibits a low pressure drop. It is also very resistant to abrasion during grinding and is therefore well suited for use in stirred tank reactors.

In contrast to the above-mentioned prior-art process of EP 0 206 687, the polyazetidine polymer is added to the solution or dispersion before dewatering, thereby avoiding the difficult mixing in a pasty state. The amount of cross-linking agent can be reduced compared to EP 0 206 687 because the mixing is more efficient. Although the polyazetidine is added prior to dewatering, very litle polyazetidine is lost during dewatering as it binds almost quantitatively to the flocculated material.

### DETAILED DESCRIPTION OF THE INVENTION

Biological materials that can be immobilized by the method of this invention include enzymes, cell mass (intact or disrupted cells, viable or non-viable), antibodies and coenzymes. The material is used as an aqueous solution or dispersion which may be purified as desired by conventional techniques. The degree of purification is not critical to the practice of the invention, but influences the properties of the immobilized product.

It has been found that the quantity of water present in the reaction mixture is not critical. Excess water will be removed during dewatering without any serious loss of active material. Thus, water may be added to obtain a convenient consistency. Conveniently, the biological material is added in the form of an aqueous dispersion or solution.

The polyazetidine polymer used for the practice of this invention may be any water-soluble polymer with a substantial content of reactive azetidine rings, such as those prepared by reacting a polyamide with epichlorohydrin according to German patent publication DT-AS-1,177,824. Examples of commercially available products are Polycup 2002, Kymene 557H and Reten 304 (these are all trade marks of Hercules Inc., USA).

The amount of polyazetidine may be used to control the properties of the immobilized product. Thus, a high amount generally yields physically strong particles, whereas a low amount yields particles with low diffusion restriction and therefore with high activity. The suitable amount will usually be in the range about 0.1 - about 10% (hereinafter, % are by weight of dry matter in the biological material), typically about 0.5 - about 5%, e.g. about 0.5 to about 3%.

pH throughout the process is generally around neutral, mostly between about 5 and about 9. Higher or lower pH may be preferred depending on enzyme stability. A buffer may be included to stabilize pH during the reaction.

Optionally, glutaraldehyde may be used as a cross-linking agent in addition to polyazetidine. The amount of glutaraldehyde may be up to about 40% (of dry matter, as above), typically below about 15%. If glutaraldehyde is used, it is preferred to hold the mixture of the biological material and glutaraldehyde to let cross-linking occur, e.g. for about 5 minutes to about 1 hour, either before or after the addition of polyazetidine.

Optionally, auxiliary cross-linking agents containing groups that react with polyazetidine, such as -NH₂ groups (e.g. polyethylene imine, chitosan, albumen or gelatine) or -COOH groups (e.g. carboxy-methyl cellulose) may be added to the reaction mixture. If glutaraldehyde is used, any -NH₂ containing agent should preferably be added prior to the glutaraldehyde. These may be used e.g. in order to increase the physical strength, to adjust the consistency of the filter cake for easy handling. Also, in some cases, it may be desirable to include these in order to dilute the activity of the immobilized material, e.g. in the case of penicillin acylase. The amount of auxiliary agent may be up to 100% of dry matter. Preferred quantities are for polyethylene imine up to 20%, for albumen or gelatine up to 50%, and for carboxy-methyl cellulose up to 10%.

Temperature during mixing (and holding, if used) is generally in the range 0-60°C. Temperature near to ambient is often convenient, but lower temperature may be needed due to enzyme instability.

It may be necessary to add a conventional flocculant before dewatering to get satisfactory flocculation. The need for this as well as suitable type and quantity of flocculant are easily determined by someone skilled in the art.

Polyazetidine can act as a cationic flocculant, besides acting as a cross-linking agent. Thus, for negatively charged materials a separate flocculant can be omitted if sufficient polyazetidine is added.

The dewatering step of the invention is intended to remove excess water after flocculation thereby generating a pasty mass. It is conveniently done by filtration or centrifugation.

Sub-dividing according to the invention is done prior to curing, as the consistency of the pasty mass at this stage permits easy forming into individual particles of controlled size. A preferred technique is extrusion. Optionally, the extruded particles may be rounded (spheronized) before or after curing, e.g. by the "Marumerizer" technique disclosed in British patent specification GB 1,362,265.

The curing step of the invention serves to remove water and to let the cross-linking reaction proceed, so that a physically strong product is obtained. Preferred techniques are air drying or fluidized-bed drying, generally at 15-80°C. In case of very sensitive biological materials, low temperature drying or freeze-drying may be needed.

Preferably, the immobilized product is dried to a water content below around 25% w/w. With an ultimate water content of above around 25% the microbial stability of the product may be unsatisfactory. Furthermore, with water content above around 25% the cross-linking with the polyazetidine prepolymer may not have taken place or may not be complete; the particles may tend to aggregate over time in storage. Drying to a water content of below about 10% may inactivate the biological material.

The process of the invention leads to an immobilized product of high physical strength, and consequently low pressure drop when used in a packed bed. The practical effect is illustrated by the fact that an immobilized product according to the invention (penicillin acylase derived from Fusarium) with a pressure drop value of 2-4 (measured as in Example 1) can be used in a fixed-bed column reactor with a height of 1 meter and a diameter of 1 meter with a holding time of 1 minute. The pressure drop over the bed remains nearly constant for more than 6 months. A similar prior-art product with a pressure drop value of more than 10 is not able to withstand these conditions.

The high physical strength of a product immobilized according to the invention also results in low grinding loss, as seen in the examples that follow. Products with low grinding loss are significantly more stable in a continuous stirred tank reactor. With penicillin acylase immobilized according to the invention, practical lifetimes of more than 4 months in 4 m³ reactor systems can be expected with activity loss of no more than 50%.

### UTILITY

The process of the invention is widely applicable to immobilization of biological materials, such as enzymes, cell mass, coenzymes and antibodies (monoclonal or polyclonal).

Enzymes may be immobilized in the form of enzymatically active cells, of partly or fully homogenized cell paste, or as a largely cell-free enzyme solution. Some examples where the method of the invention is particularly advantageous follow:
- glucose isomerase from Streptomcyes sp., e.g. from the following species:
   S. murinus
   (EP 0 194 760)
   S. flavovirens
   S. achromogenus
   S. echinatus
   S. wedmorensis
   S. albus
   (US 3,616,221)
   S. olivochromogenes
   S. venezuelae
   (US 3,622,463)
   S. griseoflavus
   (US 4,351,903)
   S. glaucescens
   US 4,137,126)
   S. olivaceus
   (US 3,625,828)
   S. galbus
   S. gracilis
   S. matensis
   S. niveus
   S. platensis
   (Hungarian patent 12,415)
   S. violaceoniger
   (German patent 2,417,642)
   S. acidodurans
   (US 4,399,222)
   S. phaeochromogenes
   S. fradiae
   S. roseochromogenes
   S. olivaceus
   S. californicus
   S. vanaceus
   S. virginiae
   (Japanese patent publication 69-28,473)
- glucose isomerase from Bacillus coagulans, (see US 3,979,261).
- glucose isomerase from Actinoplanes sp., especially A. missouriensis.
- glucoamylase from Aspergillus sp., especially from black Aspergilli and more especially from A. niger (see US patent 3,677,902), or from Rhizopus sp., especially Rh. delemar or Rh. niveus.
- penicillin-V acylase from Fusarium sp., especially F. anguioides, F. argiilaceum, F. avenaceum, F. bulbigenum, F. coeruleum, F. culmorum, F. equiseti, F. lateritium, F. minimum, F. monoliforme, F. oxysporum, F. sambucinum, F. semisectum, F. solani, and F. sulphureum (see GB 891,173).
- penicillin acylase from Eschericia coli, Proteus rettgeri, Kluyvera citrophila, Bacillus sphaericus, Bovista plumbea or Bacillus megaterium.
- lactase from Kluyveromyces sp., especially from K. fragilis or K. lactis.
- cyanide hydratase according to Danish application DK 87/1283.
- nitrilase, nitrile hydratase or amidase, especially from Rhodococcus sp., Pseudomonas sp. or Brevibacterium sp. See US 4,001,081, EP 0 093 782 and EP 0 188 316.

Cell mass to be immobilized by the process of the invention may be in the form of viable or non-viable intact cells as well as homogenized cell paste. The cells are preferably of microbial or plant origin. Some preferred examples follow:
- viable cells for use in bioconversion, e.g. yeast for ethanol fermentation.
- cells with enzymatic activity, e.g. fungal mycelium containing cyanide hydratase, see EP 0 061 249 and EP 0 116 423.
- cell mass for use in adsorptive removal, e.g. of heavy metals, see EP 0 181 497, US 4,320,093, US 4,298,334, US 4,293,333 and JP-A 49-104,454.

### EXAMPLE 1

Glucose isomerase containing cells were produced by fermentation of Streptomyces murinus, strain DSM 3253, according to European patent publication EP 0 194 760.

The cells were harvested by centrifugation of the culture broth. The cell sludge had a dry substance content of 7.0%.

The general immobilization procedure was as follows;
To 300 g cell sludge was added 300 g deionized water containing 1.5% MgSO₄,7H₂O. pH was adjusted to 7.5. The indicated amount of polyethyleneimine (Sedipur, product of BASF, West Germany) was added, and after thorough mixing the mixture was cross-linked by addition of 15% active glutaraldehyde based on cell sludge dry substance plus polyethyleneimine dry substance. After 1 hour the polyazetidine (Polycup® 2002) was added and thoroughly mixed with the cross-linked cell suspension.

The mixture was then flocculated by addition of a cationic flocculant, Superfloc C521 (Cyanamid Int.). The cross-linked enzyme was recovered by filtration, formed into particles by extrusion through a 0.8 mm screen and dried at room temperature.

The glucose isomerase activity was measured by NOVO Analysis Method F-855310 (available on request from Novo Industri A/S, Denmark) and the physical stability determined as pressure drop over a column.

The pressure drop was measured over a column with a diameter of 24 mm and an enzyme bed height of 4 cm (5 g enzyme). The solution, 45% glucose in demineralized water with 1 g MgSO₄/l, was pumped through the column at a rate of 40 g/min at 60°C. The pressure drop (in mm of liquid) describes the physical stability of the enzyme particle, i.e. a low pressure drop corresponds to a good physical stability.

The results are shown in the table below.

| | | Glucose isomerase activity (µmol/min/g) | Pressure drop (mm) |
|---|---|---|---|
| 5% polyethyleneimine | 0% polyazetidine | 614 | 400 |
| | 2.5% polyazetidine | 667 | 99 |
| | 5% polyazetidine | 586 | 57 |
| 10% polyethyleneimine | 0% polyazetidine | 692 | 105 |
| | 2.5% polyazetidine | 578 | 11 |
| | 5% polyazetidine | 506 | 10 |

Preparations with pressure drops exeeding about 20 mm liquid are considered unsuitable for industrial glucose isomerase columns, and thus the polyazetidine is essential to make an industrially useful enzyme in this example. Activity decreases slightly with increasing polyazetidine concentration.

This example clearly shows the improvement of physical stability of the immobilized preparations obtained with polyazetidine.

### EXAMPLE 2

A similar experiment to that described in Example 1 was performed using a higher yielding descendant of DSM 3253.

The dry substance content of the cell sludge was 5.3%, and the cells were partially discrupted by homogenization. Otherwise the immobilization was performed as described in Example 1.

The results are given in the table below.

| | | Glucose isomerase activity (µmol/min/g) | Pressure drop (mm) |
|---|---|---|---|
| 5% polyethyleneimine | 0% polyazetidine | 556 | 16 |
| | 1% polyazetidine | 855 | 13 |
| | 2.5% polyazetidine | 839 | 13 |
| | 5% polyazetidine | 707 | 8 |
| 10% polyethyleneimine | 0% polyazetidine | 651 | 12 |
| | 1% polyazetidine | 975 | 10 |
| | 2.5% polyazetidine | 911 | 5 |
| | 5% polyazetidine | 853 | 3 |

From these experiments, it can be concluded that the polyazetidine improves the physical stability of even very physically stable formulations.

### EXAMPLE 3

Bacillus coagulans containing glucose isomerase was immobilized with and without polyazetidine.

Homogenized cell paste of B. coagulans prepared according to US patent 3,979,261 was suspended in 0.1% MgSO₄ to a final dry substance concentration of 3%. Glutaraldehyde was added to a final concentration of 0.5%. After 60 min with mixing at room temperature polyazetidine (Polycup® 2002) was added followed by flocculation with Superfloc C521. The cross-linked enzyme was recovered by filtration, formed into particles by extrusion and dried at room temperature.

Activity and pressure drop were measured as described in Example 1. Resistance to grinding was measured as turbidity (optical density) at 600 nm after 1 hour of vigorous stirring of 0.5 g enzyme in 20 ml 50 mM phosphate, pH 7 with a propeller. Before the assay the enzyme had been swelled and washed in 6% NaCl in 50 mM phosphate, pH 7.0.

| | | | |
|---|---|---|---|
| Amount of polyazetidine (% dry substance) | 0 | 1 | 3 |
| activity (µmole/min/g) | 540 | 506 | 493 |
| pressure drop (mm liquid) | 5 | 3 | 2 |
| grinding | 0.111 | 0.068 | 0.062 |

Both resistance to grinding and physical stability were improved by adding polyazetidine, while only a small activity loss was observed.

### EXAMPLE 4

As an example of immobilization of an enzyme and not a cell mass amyloglucosidase from Aspergillus niger has been chosen. This enzyme is extracellular.

A commercial preparation AMG 400 L HP (Novo Industri A/S, Denmark) was dialyzed against 50 mM phosphate pH 7.0. The preparation was diluted to a dry substance concentration of 2%, and an equal amount of egg albumen was added. Glutaraldehyde was added to a concentration of 0.6% w/v. After one hour of stirring, polyazetidine (Polycup® 2002) was added to the indicated final concentration of 0.08% w/v, and the mixture was flocculated with Filtrafloc (Servo B.V., Netherlands). Enzyme was recovered as described in previous examples.

Activity was measured as described in Novo Analysis Method AF159/2. Pressure drop was measured as described in Example 1, but at 35°C and with 11% w/w glucose in 50 mM phosphate, pH 7.5.

| | | |
|---|---|---|
| polyazetidine, % w/v | 0 | 0.08 |
| activity (µmol/min/g)* | 196 | 160 |
| pressure drop (mm liquid) | 9 | 6 |

| | | |
|---|---|---|
| * Particle fraction: 425-710 µm | | |

### EXAMPLE 5

Cell paste of Fusarium sp. containing penicillin acylase activity (prepared according to British patent specification GB 891,173), which had been washed thoroughly with 0.9% NaCl, was suspended in 50 mM phosphate pH 7.0 to give a final dry substance content of 3%. Polyethyleneimine (Sedipur) was added to give a final dry substance content of 0.1%. Glutaraldehyde was added to a final concentration of 0.2% w/v. After one hour with thorough mixing polyazetidine was added, and finally the mixture was flocculated with a cationic flocculant, Filtrafloc. The cross-linked enzyme was recovered by filtration, formed into particles by extrusion through a 0.6 mm screen and dried at room temperature.

Enzyme activity was measured by Novo analysis AF186, and the physical stability determined as pressure drop (Example 3) and resistance to grinding (Example 3).

| Polyazetidine type 557H | None | Polycup® 2002 | | Kymene® |
|---|---|---|---|---|
| Polyazetidine (%) | 0 | 1 | 3 | 1 |
| activity* (PVU/g) | 72 | 57 | 42 | 35 |
| pressure drop (mm | 6 | 4 | 3 | 2 |
| liquid) | | | | |
| grinding | 0.563 | 0.305 | 0.140 | 0.103 |

| | | | | |
|---|---|---|---|---|
| * 450-710 µm fraction | | | | |

As can be seen from the table polyazetidine increases physical stability, i.e. gives increased resistance to grinding and gives highly improved pressure stability. However, this is obtained at the expense of an activity loss which partly is due to diffusion limitation, which again is believed to be due to a more dense preparation when polyazetidine is present.

### EXAMPLE 6

Fusarium sp. was immobilized as described in Example 5, but at different pH-values and with 1% Kymene® 557H in all preparations.

Results are seen in the table below:

| | | | |
|---|---|---|---|
| pH | 6 | 7 | 8 |
| activity (PVU/g) | 33.3 | 36.1 | 41.5 |
| pressure drop (mm liquid) | 2 | 3 | 3 |
| grinding | 0.171 | 0.154 | 0.188 |

The physical stability is good and independent of pH in the tested range (6-8).

### EXAMPLE 7

The significance of time of polyazetidine addition was examined with cell paste of Fusarium sp. Immobilization was done as explained in Example 5, but without PEI and with polyazetidine (Polycup® 2002) addition to a final concentration of 0.3% both before and after the addition of glutaraldehyde.

| Time for polyazetidine addition | not added | before glutaraldehyde | after glutaraldehyde |
|---|---|---|---|
| activity (PVU/g) | 58 | 32 | 31 |
| pressure drop (mm liquid) | 27 | 6 | 4 |

The physical stability is increased both when polyazetidine is added before and after glutaraldehyde.

### EXAMPLE 8

The effect of glutaraldehyde in combination with polyazetidine has been tested. Fusarium sp. was immobilized as described in Example 5 but without some of the components. Results are shown in the table:

| Kymene® (%) | PEI (%) | Glutaraldehyde (%) | Filtrafloc added | Activity (PVU/g) | Pressure drop (mm liquid) | Grinding resistance |
|---|---|---|---|---|---|---|
| 1 | 3 | 0 | + | 40.9 | 10 | 0.181 |
| 1 | 3 | 0 | - | 45.3 | 5 | 0.118 |
| 3 | 3 | 0 | + | 39.6 | 8 | 0.095 |
| 10 | 3 | 0 | + | 39.9 | 8 | 0.091 |
| 3 | 0 | 0 | + | 37.2 | 7 | 0.088 |
| 1 | 3 | 7 | + | 35.6 | 2 | 0.153 |

The results show that glutaraldehyde increases the physical stability, especially the resistance to pressure. Acceptable preparations can, however, be made without glutaraldehyde, PEI, or flocculating agent.

### EXAMPLE 9

Cell sludge of Rhodococcus erythropolis having ability to hydrolyze nitrile (prepared according to EP 188,316) was diluted to 2% dry substance with water. 10% polyethyleneimine (Sedipur) (based on dry substance of cell sludge) was added, and pH was adjusted to 7.0. The mixture was cross-linked by addition of 5% active glutaraldehyde (based on cell sludge dry substance plus polyethyleneimine dry substance). After 1 hour, 2% of polyazetidine (Kymene) (based on total dry substance) was added.

The mixture was then flocculated by addition of an anionic flocculant, Superfloc A 130. The cross-linked enzyme was then recovered by filtration, formed into particles by extrusion through a 0.8 mm screen and dried at room temperature.

For reference, the same cell sludge was cross-linked without polyazetidine, but otherwise in the same way as above.

For comparison, the same cell sludge was immobilized by a prior-art method, viz. entrapment in polyacrylamide gel according to US 4,248,968. More specifically, the cell sludge was diluted to 12.5% dry substance and immobilized as described in Example 12 thereof. Finally, the gel was sieved with 1 mm mesh sieve and washed with saline until the supernatant became clear.

Pressure drop was measured at the following conditions:
11% glucose syrup
- Flow rate:: 40 g/min
- Temperature:: 35°C
Pressure drop was measured after 25 hours.

5 g (dry substance) of immobilized enzyme was applied for pressure drop measurement.

Results (pressure drop in g/cm²):

### Invention:

| | |
|---|---|
| cross-linking with polyazetidine | 13 |

### Reference

| | |
|---|---|
| cross-linking without polyazetidine | 200 |
| gel entrapment | 200 |

The features disclosed in the foregoing description and in the following claims may, both separately and in any combination thereof, be material for realising the invention in diverse forms thereof.

## Claims

1. A process for immobilizing biological material by cross-linking with polyazetidine, which process comprises the sequential steps of aqueously mixing biological material with polyazetidine, flocculating the resultant mixture, dewatering the flocculated product, sub-dividing the dewatered product and then curing the sub-divided material to obtain a particle form immobilized product.

2. The process of Claim 1, wherein the amount of polyazetidine is from about 0.1% to about 10% by weight of dry matter in the biological material, preferably from about 0.5% to about 5%.

3. The process of Claim 1 or 2, wherein glutaraldehyde is incorporated in the polyazetidine/biological material mixture before dewatering is effected.

4. The process of Claim 3, wherein the amount of glutaraldehyde is below about 40% of dry matter in the biological material, preferably below about 15%.

5. The process of Claim 3 or 4, wherein the glutaraldehyde is aqueously mixed with the biological material, the glutaraldehyde/biological material mixture being then held prior to the mixing thereof with polyazetidine.

6. The process of Claim 3 or 4, wherein the glutaraldehyde and polyazetidine are aqueously mixed with the biological material, the glutaraldehyde/polyazetidine/-biological material mixture being then held prior to the dewatering thereof.

7. The process of Claims 1 - 6, further comprising admixing, preferably below about 100% dry matter, of polyethylene imine (most preferably below about 10%), albumen (most preferably below about 50%), gelatine (most preferably below about 50%) or carboxymethyl cellulose (most preferably below about 10%) before dewatering (all %'es being by weight relative to dry matter in the biological material).

8. The process of Claims 1 - 7, wherein an effective amount of a flocculant is added prior to dewatering.

9. The process of Claims 1 - 8, wherein the biological material is added in the form of an aqueous dispersion or solution.

10. The process of Claims 1 - 9, wherein the biological material comprises whole cells or cell mass from fully or partially disrupted cells, preferably of plant or microbial origin.

11. The process of Claim 10, wherein the immobilized material is characterized by being usable for adsorptive removal, preferably of heavy metals.

12. The process of Claims 1 - 10, wherein the biological material comprises an enzyme.

13. The process of Claim 12, wherein the enzyme is glucose isomerase.

14. The process of Claim 13, wherein the enzyme is derived from Streptomyces sp., most preferably S. murinus, from Bacillus coagulans or from Actinoplanes missouriensis.

15. The process of Claim 12, wherein the enzyme is penicillin acylase.

16. The process of Claim 15, wherein the enzyme is derived from Fusarium sp., preferably F. anguioides, F. argiilaceum, F. avenaceum, F. bulbigenum, F. coeruleum, F. culmorum, F. equiseti, F. lateritium, F. minimum, F. monoliforme, F. oxysporum, F. sambucinum, F. semisectum, F. solani, or F. sulphureum

17. The process of Claim 12, wherein the enzyme is nitrilase, preferably from Rhodococcus sp. (preferably Rh. erythropolis), Pseudomonas sp. or Brevibacterium sp.

18. The process of Claim 12, wherein the enzyme is cyanide hydratase.

19. The process of Claims 1 - 8, wherein the biological material is a coenzyme.

20. The process of Claims 1 - 8, wherein the biological material is an antibody.

## Patentansprüche

1. Verfahren zur Immobilisierung von biologischem Material durch Quervernetzung mit Polyazetidin, wobei dieses Verfahren die aufeinanderfolgenden Schritte umfaßt: wässeriges Vermischen von biologischem Material mit Polyazetidin, Ausflocken der resultierenden Mischung, Entwässern des ausgeflockten Produktes, Unterteilen des entwässerten Produktes und anschließend Aushärten des unterteilten Materials, um ein immobilisiertes Produkt in Teilchenform zu erhalten.

2. Verfahren nach Anspruch 1, wobei die Menge an Polyazetidin von etwa 0,1% bis etwa 10%, bezogen auf das Gewicht der Trockensubstanz im biologischen Material, vorzugsweise von etwa 0,5% bis etwa 5% beträgt.

3. Verfahren nach Anspruch 1 oder 2, wobei Glutaraldehyd in die Mischung aus Polyazetidin und biologischem Material eingearbeitet wird, bevor das Entwässern durchgeführt wird.

4. Verfahren nach Anspruch 3, wobei die Menge an Glutaraldehyd unter etwa 40% Trockensubstanz im biologischen Material, vorzugsweise unter etwa 15% liegt.

5. Verfahren nach Anspruch 3 oder 4, wobei das Glutaraldehyd mit dem biologischen Material wässerig vermischt wird, wobei die Mischung aus Glutaraldehyd und biologischem Material dann vor dem Vermischen derselben mit Polyazetidin gehalten wird.

6. Verfahren nach Anspruch 3 oder 4, wobei das Glutaraldehyd und das Polyazetidin mit dem biologischen Material wässerig vermischt werden, wobei die Mischung aus Glutaraldehyd, Polyazetidin und biologischem Material vor der Entwässern derselben gehalten wird.

7. Verfahren nach den Ansprüchen 1-6, das außerdem Zumischen, vorzugsweise unter etwa 100% Trockensubstanz, von Polyethylenimin (am bevorzugtesten unter etwa 10%), Albumin (am bevorzugtesten unter etwa 50%), Gelatine (am bevorzugtesten unter etwa 50%) oder Carboxymethylcellulose (am bevorzugtesten unter etwa 10%) vor dem Entwässern umfaßt (alle % gewichtsbezogen relativ zur Trockensubstanz im biologischen Material).

8. Verfahren nach den Ansprüchen 1-7, wobei eine wirksame Menge eines Flockungsmittels vor dem Entwässern zugegeben wird.

9. Verfahren nach den Ansprüchen 1-8, wobei das biologische Material in der Form einer wässerigen Dispersion oder Lösung zugegeben wird.

10. Verfahren nach den Ansprüchen 1-9, wobei das biologische Material ganze Zellen oder Zellmasse aus vollständig oder teilweise aufgebrochenen Zellen, vorzugsweise mit pflanzlichem oder mikrobiellem Ursprung, umfaßt.

11. Verfahren nach Anspruch 10, wobei das immobilisierte Material dadurch gekennzeichnet ist, daß es für adsorptive Entfernung, vorzugsweise von Schwermetallen verwendbar ist.

12. Verfahren nach den Ansprüchen 1-10, wobei das biologische Material ein Enzym umfaßt.

13. Verfahren nach Anspruch 12, wobei das Enzym Glucose-Isomerase ist.

14. Verfahren nach Anspruch 13, wobei das Enzym gewonnen ist aus Streptomyces sp., am bevorzugtesten S. murinus, aus Bacillus coagulans oder aus Actinoplanes missouriensis.

15. Verfahren nach Anspruch 12, wobei das Enzym Penicillin-Acylase ist.

16. Verfahren nach Anspruch 15, wobei das Enzym gewonnen ist aus Fusarium sp., vorzugsweise F. anguioides, F. argiilaceum, F. avenaceum, F. bulbigenum, F. coeruleum, F. culmorum, F. equiseti, F. lateritium, F. minimum, F. monoliforme, F. oxysporum, F. sambucinum, F. semisectum, F. solani oder F. sulphureum.

17. Verfahren nach Anspruch 12, wobei das Enzym Nitrilase ist, vorzugsweise aus Rhodococcus sp. (vorzugsweise Rh. erythronolis), Pseudomonas sp. oder Brevibacterium sp.

18. Verfahren nach Anspruch 12, wobei das Enzym Cyanid-Hydratase ist.

19. Verfahren nach den Ansprüchen 1-8, wobei das biologische Material Coenzym ist.

20. Verfahren nach den Ansprüchen 1-8, wobei das biologische Material ein Antikörper ist.

## Revendications

1. Procédé pour immobiliser une substance biologique par réticulation avec la polyazétidine, ce procédé comprenant les étapes successives de mélange de la substance biologique avec la polyazétidine en milieu aqueux, de floculation du mélange résultant, de déshydratation du produit floculé, de subdivision du produit déshydraté et de maturation du produit subdivisé pour obtenir un produit immobilisé sous forme de particules.

2. Procédé selon la revendication 1, dans lequel la quantité de polyazétidine est d'environ 0,1% à environ 10% en poids de la matière sèche dans la substance biologique, de préférence d'environ 0,5% à environ 5%.

3. Procédé selon la revendication 1 ou 2, dans lequel du glutaraldéhyde est incorporé dans le mélange polyazétidine/substance biologique avant la déshydratation.

4. Procédé selon la revendication 3, dans lequel la quantité de glutaraldéhyde est inférieure à environ 40% de la matière sèche dans la substance biologique, de préférence inférieure à environ 15%.

5. Procédé selon la revendication 3 ou 4, dans lequel le glutaraldéhyde est mélangé en milieu aqueux avec la substance biologique, le mélange glutaraldéhyde/substance biologique étant ensuite conservé avant son mélange avec la polyazétidine.

6. Procédé selon la revendication 3 ou 4, dans lequel le glutaraldéhyde et la polyazétidine sont mélangés en milieu aqueux avec la substance biologique, le mélange glutaraldéhyde/polyazétidine/substance biologique étant ensuite conservé avant sa déshydratation.

7. Procédé selon les revendications 1 à 6, comprenant en outre le mélange, de préférence en une quantité inférieure à environ 100% de matière sèche, de polyéthylénimine (de manière particulièrement préférée en une quantité inférieure à environ 10%), d'albumine (de manière particulièrement préférée en une quantité inférieure à environ 50%), de gélatine (de manière particulièrement préférée en une quantité inférieure à environ 50%) ou de carboxyméthylcellulose (de manière particulièrement préférée en une quantité inférieure à environ 10%) avant la déshydratation (tous les pourcentages étant en poids par rapport à la matière sèche dans la substance biologique).

8. Procédé selon les revendications 1 à 7, dans lequel une quantité efficace de floculant est ajoutée avant la déshydratation.

9. Procédé selon les revendications 1 à 8, dans lequel la substance biologique est ajoutée sous forme d'une dispersion ou solution aqueuse.

10. Procédé selon les revendications 1 à 9, dans lequel la substance biologique comprend des cellules entières ou une masse de cellules provenant de cellules totalement ou partiellement lysées, de préférence d'origine végétale ou microbienne.

11. Procédé selon la revendication 10, dans lequel la substance immobilisée est caractérisée par le fait qu'elle est utilisable pour le retrait par adsorption, de préférence de métaux lourds.

12. Procédé selon les revendications 1 à 10, dans lequel la substance biologique comprend une enzyme.

13. Procédé selon la revendication 12, dans lequel l'enzyme est la glucose isomérase.

14. Procédé selon la revendication 13, dans lequel l'enzyme provient de Streptomyces sp., de préférence encore de S. murinus, de Bacillus coagulans ou d'Actinoplanes missouriensis.

15. Procédé selon la revendication 12, dans lequel l'enzyme est la pénicilline acylase.

16. Procédé selon la revendication 15, dans lequel l'enzyme provient de Fusarium sp., de préférence de F. anguioides, de F. argiilaceum, de F. avenaceum, de F. bulbigenum, de F. coeruleum, de F. culmorum, de F. equiseti, de F. lateritium, de F. minimum, de F. monoliforme, de F. oxysporum, de F. sambucinum, de F. semisectum, de F. solani ou de F. sulphureum.

17. Procédé selon la revendication 12, dans lequel l'enzyme est une nitrilase, provenant de préférence de Rhodococcus sp. (de préférence de Rh. erythropolis), de Pseudomonas sp. ou de Brevibacterium sp.

18. Procédé selon la revendication 12, dans lequel l'enzyme est la cyanure hydratase.

19. Procédé selon les revendications 1 à 8, dans lequel la substance biologique est une co-enzyme.

20. Procédé selon les revendications 1 à 8, dans lequel la substance biologique est un anticorps.
